Europäisches Patentamt

European Patent Office

Office européen des brevets

(11) Veröffentlichungsnummer: **0 216 152 B1**

# (12) EUROPÄISCHE PATENTSCHRIFT

(45) Veröffentlichungstag der Patentschrift: **27.12.91**

(21) Anmeldenummer: **86111556.6**

(22) Anmeldetag: **21.08.86**

(51) Int. Cl.5: **B01J 8/02**, C01C 1/04, C07C 29/15, C01B 3/02

(54) Stehender Reaktor für katalytische exotherme und endotherme Reaktionen.

(30) Priorität: **27.09.85 DE 3534402**
**22.02.86 DE 3605792**

(43) Veröffentlichungstag der Anmeldung:
**01.04.87 Patentblatt 87/14**

(45) Bekanntmachung des Hinweises auf die
Patenterteilung:
**27.12.91 Patentblatt 91/52**

(84) Benannte Vertragsstaaten:
**AT DE FR GB IT NL SE**

(56) Entgegenhaltungen:
**EP-A- 0 082 609**
**DE-A- 3 334 775**
**US-A- 3 796 547**

(73) Patentinhaber: **MAN Gutehoffnungshütte Aktiengesellschaft**
**Bahnhofstrasse 66 Postfach 11 02 40**
**W-4200 Oberhausen 11(DE)**

(72) Erfinder: **Vollhardt, Frohmut, Dipl.-Ing.**
**Nettelbeckstrasse 2**
**W-4200 Oberhausen 11(DE)**
Erfinder: **Krämer, Hans-Dieter, Dipl.-Ing.**
**Amalienstrasse 52**
**W-4220 Dinslaken(DE)**

**Beschreibung**

Die Erfindung betrifft einen stehenden Reaktor für katalytische exotherme und endotherme Reaktionen, insbesondere zur Herstellung von Methanol, Ammoniak, Synthesegas und höheren Alkoholen nach dem Oberbegriff des Hauptanspruches.

Durch die DE-A- 33 32 049 ist ein Reaktor für katalytische exotherme Reaktionen, insbesondere zur Herstellung von Methanol, bekannt. Bei ihm erstreckt sich von einer oberen Sammelringleitung zu einer unteren Verteilerringleitung ein Flossenrohrmantel, dessen Rohre im oberen und unteren Abschnitt des Reaktors als verstärkte Rohrabschnitte ausgebildet sind. In diese münden sog. Henkelrohre, deren vertikale Abschnitte Gassen bilden, in denen die Reaktormasse Platz findet, die auf einem Netz im unteren Bereich des Ofens aufsitzt.

Durch die DE-A- 33 34 775 ist ein Reaktor für katalytische exotherme Reaktionen zur Herstellung von Methanol bekannt, bei dem die oberen und unteren Enden gerader oder im wesentlichen gerader Austauscherrohre des Rohrpaketes in unterhalb und oberhalb der Sammel-bzw. Verteilerrohre angeordnete horizontale Tragrohre münden, wobei in die Kopf- und Fußrohre weitere senkrechte gerade Tauscherrohre je eines Rohrpaketes münden. Diese Tragrohre sind über Zwischen- oder Verbindungsrohre mit den Sammel- oder Verteilerrohren verbunden.

Für das Auswechseln der mit den Tragrohren verbundenen Tauscherrohre ist das Abtrennen jedes Tauscherrohres von dem Tragrohr oder des Tragrohres mit allen an diesem befestigten Tauscherrohren von dem oberen und unteren Sammel- und Verteilerrohr notwendig.

Gegenüber den bekannten Reaktoren besteht die Aufgabe der Erfindung darin, eine billigere und konstruktiv einfachere Ausbildung des Tauscherrohrpakets zu schaffen, bei dem eine statisch vorteilhaftere Verbindung der Austauscherrohre mit den Tragrohren und eine bessere Reparaturmöglichkeit, insbesondere an den Einschweißstellen der Rohre, gegeben ist. Diese Aufgabe umfaßt mit dem Tauscherrohrpaket selbstverständlich auch die konstruktive Ausbildung des Katalysatorbodens. Für den Boden und die Bodenauslässe gilt es, eine konstruktiv einfache und funktionelle, zuverlässige Ausbildung zu schaffen. Der Raum außerhalb der Austauscherrohre des Reaktors soll vollständig mit Katalysatormasse aufgefüllt werden können und der Abzug der unbrauchbar gewordenen Katalysatormasse soll auf einfache und schnelle Weise vonstatten gehen können, ohne daß gealterte Katalysatormasse in den Austauscherrohrgassen hängen bleibt.

Zur Lösung dieser Aufgabe sieht die Erfindung die Merkmale des kennzeichnenden Teils des Hauptanspruchs vor. Die Merkmale der Unteransprüche dienen der Verbesserung und Weiterentwicklung der Merkmale des Hauptanspruches.

Bei dem bekannten, den Gattungsbegriff bestimmenden Reaktor übten die Henkelrohre ein Moment auf die Rohre aus. Ferner war es bei diesen Rohren schwierig, an die Schweißstellen der nahe beieinanderliegenden Einmündungen der horizontalen Schenkel der Henkelrohre in die Verstärkungen der Flossenrohre des Mantels heranzugelangen. Bei Reparatur nur einiger Austauscherrohre bedurfte es des Heraushebens des gesamten Rohrpaketes aus dem Flossenrohrmantel. Die Austauscherrohre des erfindungsgemäßen Reaktors dagegen üben auf die sie tragenden und mit ihnen verbundenen Teile ausschließlich eine vertikale Kraft aus; die Verbindungsstellen der Austauscherrohre mit den Tragrohren sind leicht von oben oder unten zugänglich, wie auch das Auswechseln mehrerer Austauscherrohre möglich ist, ohne das gesamte Rohrpaket aus dem Reaktor herausheben zu müssen. Auch läßt sich das Abziehen der Reaktormasse aus dem durch eine ringförmige Schürze umschlossenen Innenraum des Reaktors, der die Austauscherrohre aufnimmt, leicht und gezielt vornehmen.

Auf der Zeichnung ist ein Ausführungsbeispiel eines Reaktors nach der Erfindung zur Durchführung einer exothermen Reaktion, z. B. für die Herstellung von Methanol, dargestellt und zwar zeigt

| | |
|---|---|
| Fig. 1 | einen vertikalen Längsschnitt durch den Reaktor in zwei zueinander senkrechten Ebenen, |
| Fig. 2 | einen horizontalen Querschnitt durch den oberen Abschnitt des Reaktors, |
| Fig. 3 | einen Querschnitt durch den mittleren und |
| Fig. 4 | einen horizontalen Querschnitt durch den unteren Abschnitt des Reaktors, |
| Fig. 5 und 6 | in vergrößertem Maßstab gegenüber Fig. 1 den unteren Reaktorbereich mit dem die Reaktormasse tragenden Boden und |
| Fig. 7 | eine Besonderheit dieses Bodens. |

Der Reaktor 1 weist einen zylindrischen Druckmantel 2 auf, an den sich nach oben die Haube 3 und nach unten der gleichfalls gewölbte Boden 4 anschließen. Durch Durchführungsstücke 5 ragen Abführrohre 6 für das erwärmte Medium in das Innere des Reaktors 1 und münden dort in zwei horizontale gewölbte Sammelrohre 7, 8. Im dargestellten Beispiel sind die beiden Rohre 7, 8 symmetrisch zur Quermittelebene Q des Reaktors angeordnet.

Von den gekrümmten Sammelrohren 7, 8 gehen Zwischenrohre 9, 10, 11, 12 ab, die z. B. zu viert in einer gemeinsamen Ebene liegen und paarweise symmetrisch zur senkrechten Mittelebene 13 der Rohre 7, 8 angeordnet sind sowie in gleichmäßigem Abstand in Tragrohre 14 münden. Diese erstrecken sich in horizontaler Ebene und gleichem Abstand parallel zueinander.

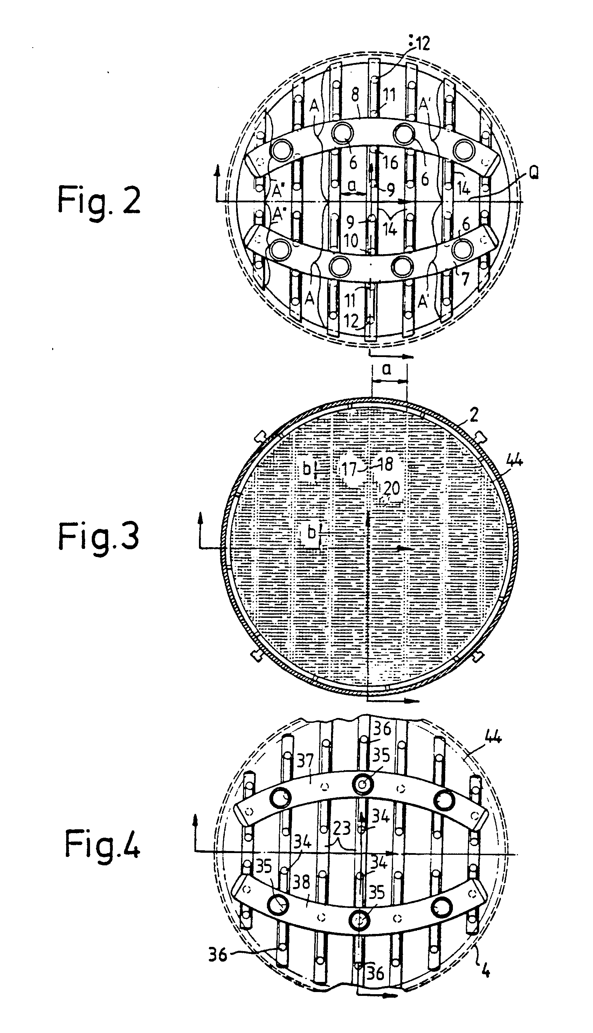

Statt der beiden Sammelrohre 7, 8 ist es möglich, in der Quermittelebene Q ein einziges Rohr vorzusehen, von dem aus wieder Zwischenrohre, ähnlich den Zwischenrohren 9 bis 12, ausgehen, die in die Tragrohre 14 münden. Hierbei jedoch ist zu berücksichtigen, daß eine solche Ausbildung hohe Anforderungen an ein solches mittleres Sammelrohr stellt, an dem, wie unten noch näher beschrieben wird, das gesamte Paket aus Wärmetauscherrohren hängt. - Es ist daher die Unterteilung eines solchen einzigen Rohres in die beiden, in Fig. 2 dargestellten gekrümmten Tragrohre 7, 8 vorteilhaft, die ihrerseits symmetrisch zur Mittelebene Q angeordnet sind und sich über die Mitte der Rohrhälften der Rohre 6 erstrecken, d. h. sich in der Mitte oberhalb der hälftigen Rohrlängen A, A' und A'' z. B. erstrecken (Fig. 2 ).

Hierbei münden die Zwischenrohre 9 bis 12 im unteren und mittleren Bereich der Sammelrohre 7, 8 in diese.

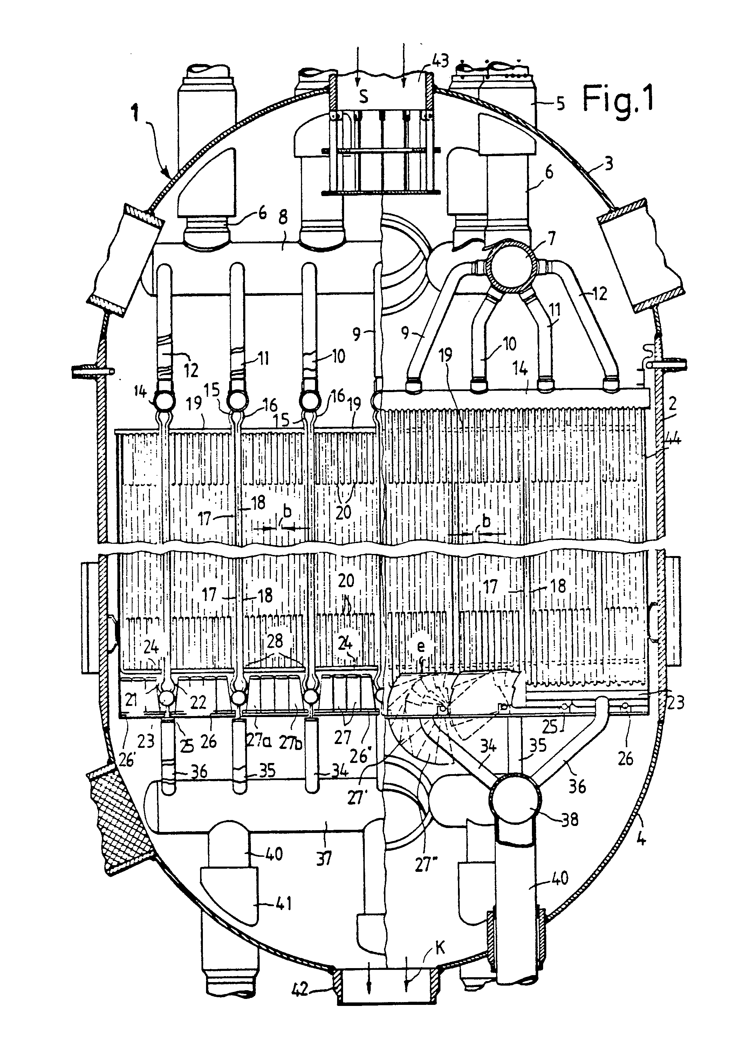

In den unteren Bereich der oberen Tragrohre 14 münden die Enden 15, 16 von Wärmetauscherrohren 17, 18. Hierbei sind die Enden 15, 16, wie Fig. 1 erkennen läßt, nach außen ausgebaucht, wodurch eine vorteilhafte Verbindung der Rohre 17, 18 mit den Tragrohren 14 erfolgen kann. Die als Haupttragaustauscherrohre zu bezeichnenden Rohre 17, 18 sind einerseits im Abstand a angeordnet, andererseits sind sie in Längsrichtung der Tragrohre im Abstand b mit diesen verbunden. - Zwischen dem oberen Abschnitt im Abstand a benachbarter Rohre 17, 18 erstrecken sich kurz unterhalb der seitlichen Ausbauchung Kopfrohre 19, an denen weitere Austauscherrohre 20 im Abstand b hängen, die parallel zu den Rohren 17, 18 verlaufen, so daß, wie Fig. 3 erkennen läßt, im horizontalen Querschnitt des Reaktormittelabschnitts ein geschlossenes Feld von parallelen senkrechten Austauscherrohren 17, 18 und 20 gebildet wird, die gleichen Abstand voneinander haben und zwischen denen die Katalysatormasse Platz findet.

Das untere Ende 21, 22 der Tragaustauscherrohre 17, 18 mündet in untere Tragrohre 23, die die gleiche Anordnung besitzen wie die oberen Tragrohre 14. Die Enden 21, 22 der Tragaustauscherrohre 17, 18 sind hierbei in gleicher Weise ausgebildet wie die oberen Enden 15, 16 dieser Rohre, wie auch zwischen im Abstand a benachbarten Rohren 21, 22 ein Fußrohr 24 vorgesehen ist, in das die unteren Enden der Austauscherrohre 20 münden. - Statt der seitlichen Ausbauchungen der oberen und unteren Enden der Rohre 17, 18, können die genannten Enden auch über Hosenstücke in die Tragrohre 14, 23 münden.

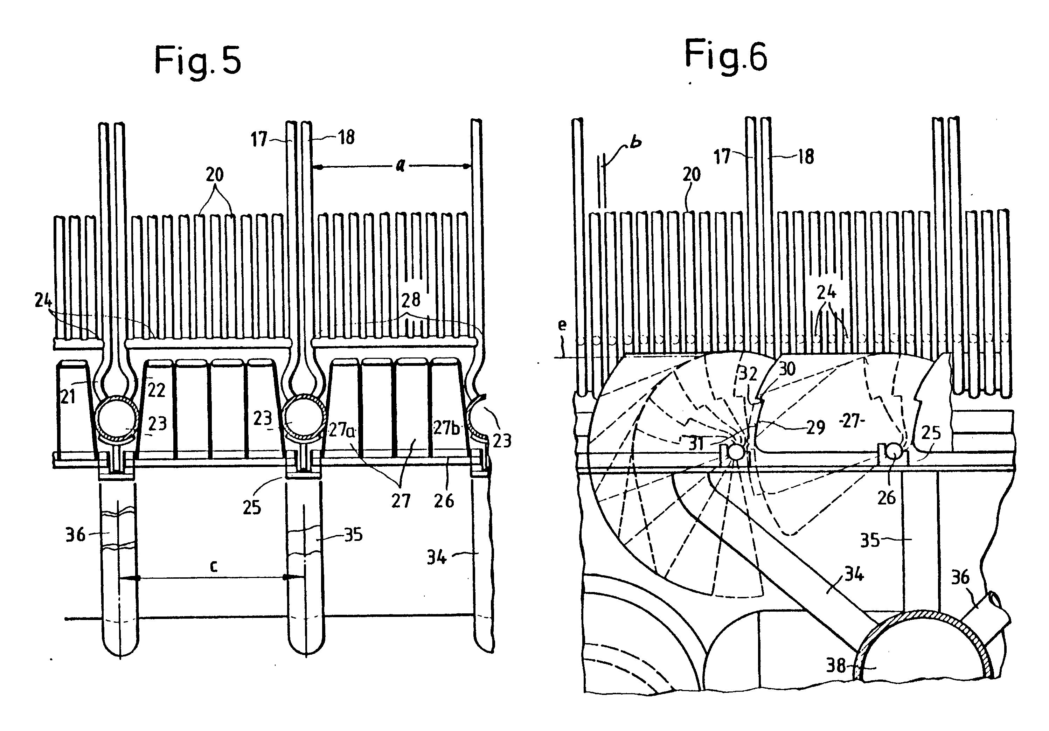

Die unteren, parallel zu den oberen Tragrohren 14 verlaufenden unteren Tragrohre 23 weisen an ihrer Unterseite Lageransätze 25 auf, die in Querrichtung der Rohre 23 miteinander fluchten und zu mehreren über die Länge jedes Tragrohres 23 angeordnet sind. Zwischen benachbarten, miteinander fluchtenden Ansätzen 25 sind Achsen 26 angeordnet, und denen nebeneinander Elemente 27 befestigt sind, die in Gruppen - in dargestelltem Beispiel zu viert -, einen Abschnitt 28 des Katalysatorbodens bilden, wobei dieser Abschnitt in hochgeklappter Wirkstellung 27 (Fig. 5 und 6 in ausgezogener Linie) in einer Ebene e liegt. Die Elemente 27 der Abschnitte 28, die den Katalysatorboden bilden, sind gasdurchlässig. Die jeweils äßeren Elemente 27a, 27b eines Abschnitts 28 liegen in hochgeklapptem Zustand der Abschnitte an den Rohren 23 an. - Die Katalysatormasse ruht auf den Abschnitten 28, die in der Draufsicht ein Quadrat mit einer Kantenlänge bilden, die etwas geringer als der Abstand a ist.

Die der Achse 26 benachbarte Rückseite 29 weist einen Rücksprung 30 auf, während die der Achse 26 abgekehrte Vorderseite 31 jedes Elementes 27 einen nasenartigen Vorsprung 32 trägt. Die Ausbildung und Anordnung des Vorsprunges 32 und des Rücksprunges 30 ist so getroffen, daß in der hochgeklappten Wirkstellung der Abschnitte 28 diese sich durch Ineinandergreifen benachbarter Vor- und Rücksprünge in ihrer Lage halten, wobei der letzte Abschnitt jeder Reihe (in Fig. 1 der am linken Zeichnungsrand) zu denkender Abschnitt festgelegt ist. Wird dieser gelöst und schwenkt nach unten, so kommt zunächst der Vor- und Rücksprung des diesem ersten Abschnitt benachbarten Abschnitts außer Eingriff, so daß dieser zwischen die Rohre 23 nach unten schwenkt und dies alle übrigen Abschnitte tun, deren Achsen miteinander fluchten, so z. B. die Achsen 26', 26'', 26''' in Fig. 5 usw.

Die unteren Tragrohre 23 sind über Zwischenrohre 34, 35, 36 mit zwei Verteilerrohren 37, 38 verbunden, die symmetrisch zur Quermittelebene Q angeordnet sind und in die Hälfte aller Zwischenrohre 34, 35, 36 münden. Die Verteilerrohre sind mit den Zuführungsrohren 40 verbunden, die jeweils durch ein Durchführungsstück 41 des Bodens 4 des Reaktors geführt sind. Dieser weist den Austrittsstutzen 42 für das in Pfeilrichtung K zuströmende Medium auf, das durch den Stutzen 43 der Haube 3 in Pfeilrichtung S in den Reaktor eintritt.

Das Austauscherrohrpaket aus den Rohren 17 bis 20 ist von einer Schürze 44 umschlossen.

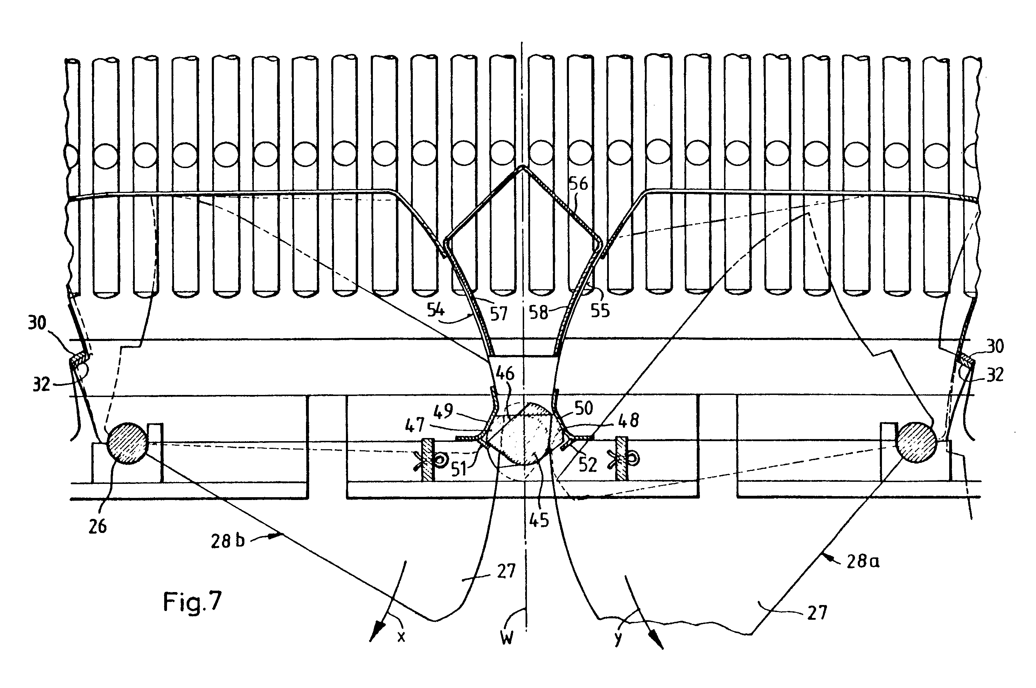

In Fig. 7 ist der mittlere Abschnitt des unteren, den Katalysatorboden aufnehmenden Bereichs des Reaktors dargestellt, wie er vorteilhafterweise bei Anwendung der vorstehend beschriebenen schwenkbaren Bodenabschnitte 28 ausgebildet werden kann. Parallel zu den Einzelwellen 26 erstreckt sich in der senkrechten Reaktormittelebene W über den gesamten Reaktorquerschnitt eine horizontale durchgehende Welle 45, die durch nicht dargestellte Mittel drehbar ist und mindestens im Abstand der einzelnen Bodenabschnitte 28 voneinander Stützkörper 46 trägt, die drehfest mit der Welle 45 verbunden sind. Diese Stützkörper weisen mit ihrer symmetrisch zur senkrechten Mittelachse der Welle 45 und der Reaktormittelebene W zwei Stützflächen 47, 48 auf, auf denen sich Stützflächen 49, 50 der einander zugekehrten Elemente 27 der mittleren Bodenabschnitte 28 abstützen, wobei die Anordnung der Abschnitte so getroffen ist, daß sich diese beidseitig der Ebene W in entgegengesetzter Richtung verschwenken lassen. (Vgl. Pfeile x, y in Fig. 7). Die Welle 45 mit den Stützflächen stellt eine Art Waagebalken dar. - Beim Drehen der Welle 45 z. B. entgegen dem Uhrzeigersinn wird zunächst der rechte Abschnitt 28a frei. Beim Weiterdrehen kommt dann auch der Bodenabschnitt 28b frei. - Klemmen die Bodenabschnitte 28 z. B. in folge Backens der Katalysatormasse, so stoßen die entsprechenden Randbereiche der Stützkörper 46 gegen Ansätze oder Nocken 51, 52 am unteren Ende der Stützflächen 49, 50 der Elemente 27 bzw. der Abschnitte 28. Zum Öffnen des jeweils zweiten Abschnitts 28 ist hierbei ein Drehen der Welle 45 um 90° notwendig.

Der Bereich 53 zwischen den geschwungenen Vorderseiten 54, 55 der in der Reaktormitte einander benachbarten Abschnitte 28a, 28b ist durch einen dachartigen Teil 56 abgedeckt, dessen Schenkelteile 57, 58 den Vorderseiten 54, 55 der Elemente 27 angepaßt sind.

Zur Durchführung einer endothermen Reaktion werden die Austauscherrohre 17, 18 mit einem Heizmedium beaufschlagt; die Sammel- und Verteilerrohre 6, 7 bzw. 37, 38 sind mit entsprechenden Zu- und Ableitungen verbunden.

## Patentansprüche

1. Stehender Reaktor für katalytische exotherme und endotherme Reaktionen, insbesondere zur Herstellung von Methanol, Ammoniak, Synthesegas und höheren Alkoholen, mit einem das Katalysatorbett aufnehmenden Gehäuse und das Gehäuse parallel zu dessen Längsachse durchsetzenden, Rohrpakete bildenden Austauscherrohre, mit einem das Katalysatorbett tragenden, gasdurchlässigen Boden sowie mit die Gehäusehaube und den Gehäuseboden durchsetzenden Zu- und Abführrohren für das Kühl- bzw. Heizmedium, die in horizontale Verteiler- und Sammelrohre münden, sowie mit Zu- und Ableitungen für das Reaktionsgas, wobei die oberen und unteren Enden gerader oder im wesentlichen gerader Austauscherrohre jedes Rohrpakets in unterhalb und oberhalb der Sammel- bzw. Verteilerrohre angeordnete, zueinander parallele horizontale Tragrohre münden, die über Zwischenrohre mit den Sammel- oder Verteilerrohren verbunden sind, dadurch gekennzeichnet, daß unter- bzw. oberhalb der Tragrohre (14, 23) horizontale Kopf- und Fußrohre (19, 24) angeordnet sind, die in die mit den Tragrohren verbundenen Austauscherrohre (17, 18) münden, wobei in die Kopf- und Fußrohre (19, 24) weitere senkrechte gerade Tauscherrohre (20) je eines Rohrpaketes münden und daß der von dem Rohrpaket getragene gasdurchlässige Katalysatorboden aus mehreren einzeln verstellbaren Bodenabschnitten (28) gebildet ist.

2. Reaktor nach Anspruch 1, dadurch gekennzeichnet, daß die in die Tragohre (14, 23) mündenden Austauscherrohre (17, 18) jedes Rohrpakets unter Bildung einer seitlichen Ausbauchung (15, 16; 21, 22) paarweise in die Tragrohre (14, 23) münden, wobei die Ausbauchungen eines Rohrpaares (17, 18) in einer rechtwinklig zur Längsachse der Tragrohre (14, 23) gelegenen Ebene voneinander fortgerichtet sind.

3. Reaktor nach Anspruch 1, dadurch gekennzeichnet, daß die oberen und unteren Enden der Rohre (17, 18) paarweise über Hosenstückrohre in die Tragrohre (14, 23) münden.

4. Reaktor nach Anspruch 1 und einem der Ansprüche 2 oder 3, dadurch gekennzeichnet, daß die Kopf- und Fußrohre (19, 24) unmittelbar unterhalb bzw. oberhalb der seitlichen Ausbauchungen (15, 16; 21, 22) der mit den Tragrohren (14, 23) verbundenen Austauscherrohre (17, 18) in diese münden.

5. Reaktor nach Anspruch 1 und einem der Ansprüche 2 bis 4, dadurch gekennzeichnet, daß die Sammel- und Verteilerrohre (7, 8 bzw. 37, 38) gekrümmt ausgebildet sind und je ein Rohr (7, 8 bzw. 37, 38) in einer Querschnittshälfte des Reaktors (1) angeordnet ist, wobei die Rohre (7, 8 bzw. 37, 38) symmetrisch zur Quermittelebene des Reaktors angeordnet sind.

**6.** Reaktor nach Anspruch 1,
dadurch gekennzeichnet,
daß die Bodenabschnitte (28) aus Einzelelementen (27) bestehen, die mit Hilfe von an den unteren Tragrohren (23) gelagerten Einzelwellen (26) schwenkbar sind.

**7.** Reaktor nach Anspruch 6,
dadurch gekennzeichnet,
daß die Vorder- und Rückseite (29, 31) der Bodeneinzelelemente (27) mit hakenförmigen Vorsprüngen (32) und entsprechenden Rücksprüngen (30) versehen sind, die bei geschlossenem Boden ineinandergreifen.

**8.** Reaktor nach Ansprüchen 6 und 7,
dadurch gekennzeichnet,
daß die beidseitig der senkrechten Reaktormittelebene (W) gelegenen Bodenabschnitte (28a, 28b) des Rohrbodens in entgegengesetzter Richtung (x, y) schwenkbar sind.

**9.** Reaktor nach Anspruch 8,
dadurch gekennzeichnet,
daß zwischen den beiden beiderseits der senkrechten Reaktormittelebene (W) und parallel zu den Einzelwellen (26) der Abschnitte (28) angeordnete Bodenabschnitte (28a, 28b) sich auf gemeinsame schwenkbare Stützkörper (46) abstützen.

**10.** Reaktor nach Anspruch 9,
dadurch gekennzeichnet,
daß die mindestens im Abstand der Bodenabschnitte (28a, 28b) zu jeder Seite der Reaktormittelebene (W) angeordneten Stützkörper (46) durch eine gemeinsame Welle (45) in der senkrechten Reaktormittelebene schwenkbar sind und Stützflächen (47, 48) aufweisen, auf denen Stützflächen (49, 50) der Bodenabschnitte (28a, 28b) aufliegen.

**11.** Reaktor nach Anspruch 10,
dadurch gekennzeichnet,
daß das untere Ende der Stützflächen (49, 50) der Bodenabschnitte (28a, 28b) mit Ansätzen oder Nocken (51, 52) versehen sind.

**12.** Reaktor nach Anspruch 8 - 11,
dadurch gekennzeichnet,
daß die Welle (45) zwischen den Bodenabschnitten (28a, 28b) von einem dachartigen Teil (56) überdeckt ist.

## Claims

**1.** Stationary reactor for catalytic exothermic and endothermic reactions, in particular for the production of methanol, ammonia, synthesis gas and higher alcohols, with a housing which accomodates the catalysor bed and with exchanger pipes which penetrate the housing parallel to the longitudial axis thereof and form pipe groups, with a gas-permeable base which supports the catalysor bed and with delivery and discharge pipes for the cooling or heating medium, these pipes penetrating the housing hood and the housing base and leading into horizontal delivery and discharge pipes for the reaction gas, wherein the upper and lower ends of the straight or essentially straight exchanger pipes of each pipe group lead into horizontal carrier pipes which are located below and above the delivery or discharge pipes, are parallel to one another, and are connected by intermediate pipes to the delivery and discharge pipes, characterised in that horizontal header and footer pipes (19, 24) are located below and above the carrier pipes (14, 23) respectively, these header and footer pipes leading into the exchanger pipes (17, 18), wherein further perpendicular straight exchanger pipes (20) of each pipe group lead into the header and footer pipes (19, 24); and in that the gas-permeable catalysor base supported by the pipe group is formed of a plurality of individual adjustable base parts (28).

**2.** Reactor according to claim 1, characterised in that the exchanger pipes (17, 18) of each pipe group which lead into the carrier pipes (14, 23) in pairs, forming a lateral widening (15, 16; 21, 22), wherein the widenings of a pair of pipes (17, 18) extend in a plane that is at right-angles to the longitudinal axis of the carrier pipes (14, 23).

**3.** Reactor according to claim 1, characterised in that the upper and lower ends of the pipes (17, 18) lead via bifurcated pipes in pairs into the carrier pipes (14, 23).

**4.** Reactor according to claim 1 and one of claims 2 or 3, characterised in that the header and footer pipes (19, 24), immediately below or above the lateral widenings (15, 16; 21, 22) of the exchanger pipes (17, 18) connected to the carrier pipes (14, 23), lead into the exchanger pipes.

**5.** Reactor according to claim 1 and one of claims 2 to 4, characterised in that the delivery and discharge pipes (7, 8 and 37,38) are formed in a bent manner and one pipe (7, 8 or 37, 38) is located in each case in a cross-sectional half of the reactor (1) wherein the pipes (7, 8 or 37, 38) are located symmetrically to the cross-sectional plane of the reactor.

6. Reactor according to claim 1, characterised in that the base sections (28) consist of individual elements (27) which can be pivoted by means of individual shafts (26) which are mounted on the lower carrier pipes (23).

7. Reactor according to claim 6, characterised in that the front and rear sides (29, 31) of the individual base parts (27) are provided with hook-shaped projections (32) and corresponding counter-projections (30), which engage in one another when the base is closed.

8. Reactor according to claims 6 and 7, characterised in that the base pieces (28a, 28b) of the pipe base which are located on either side of the vertical central plane (W) of the reactor, can be pivoted in opposite directions (x, y).

9. Reactor according to claim 8, characterised in that, between the two base parts (28a, 28b) which are disposed either side of the vertical central plane (W) of the reactor and parallel to the individual shafts (26) of the sections (28), are supported on the common pivotable supporting bodies (46).

10. Reactor according to claim 9, characterised in that the supporting bodies (46) which are disposed at least at a distance from the base sections (28a, 28b) on either side of the central plane (W) of the reactor can be pivoted in the vertical central plane of the reactor by a common shaft (45) and have supporting surfaces (47, 48) on which supporting surfaces (49, 50) of the base parts (28a,28b) rest.

11. Reactor according to claim 1,characterised in that the lower end of the supporting surfaces (49, 50) of the base sections (28a, 28b) are provided with attachments or cams (51, 52).

12. Reactor according to claims 8 - 11, characterised in that the shaft (45) is covered by a roof-like part (56) between the base sections (28a, 28b).

## Revendications

1. Réacteur vertical pour réactions catalytiques exothermiques et endothermiques, en particulier pour la fabrication de méthanol, ammoniac, gaz de synthèse et alcools supérieurs, avec un carter recevant le lit du catalyseur et des tubes d'échangeur constituant des paquets de tubes, traversant le carter parallèlement à son axe longitudinal, avec un fond, laissant passer les gaz, portant le lit du catalyseur ainsi qu'avec des tubes d'amenée et d'évacuation, traversant le dôme et le fond du carter, pour l'agent de refroidissement ou de chauffage, qui débouchent dans les collecteurs et répartiteurs horizontaux, ainsi qu'avec des conduites d'amenée et d'évacuation pour les gaz de la réaction, dans lequel les extrémités supérieures et inférieures des tubes de l'échangeur rectilignes ou sensiblement rectilignes de chaque paquet de tubes débouchent dans des tubes porteurs disposés en-dessous et au-dessus des collecteurs ou répartiteurs, et placés horizontalement parallèlement les uns aux autres, tubes porteurs qui son reliés par des tubes intermédiaires aux collecteurs ou répartiteurs, réacteur caractérisé en ce que en-dessous ou au-dessus des tubes porteurs (14,23) son disposés des tubes horizontaux de tête et de base (19,24), qui débouchent dans les tubes d'échangeur (17,18) reliés aux tubes porteurs, d'autres tubes rectilignes verticaux (20) de chacun des paquets de tubes débouchent dans les tubes de tête et de base (17,18) et en ce que le fond du catalyseur supporté par le paquet de tubes et perméable aux gaz est formé de plusieurs sections (28) de fond pouvant individuellement se mouvoir.

2. Réacteur selon la revendication 1, caractérisé en ce que les tubes d'échangeur (17,18) de chaque paquet de tubes débouchant dans les tubes porteurs (14,23) débouchent par paire dans les tubes porteurs (14,23) grâce à la formation d'un évasement latéral (15,16,21), les évasements d'une paire de tubes (17,18) sont espacés les uns des autres dans un plan situé à angle droit par rapport à l'axe longitudinal des tubes porteurs (14,23).

3. Réacteur selon la revendication 1, caractérisé en ce que les extrémités supérieures et inférieures des tubes (17,18) débouchent par paire dans les tubes porteurs (14,23) par l'intermédiaire de culasses.

4. Réacteur selon la revendication 1, et l'une des revendications 2 ou 3, caractérisé en ce que les tubes de tête et de pied (19,24) débouchent diversement en-dessous ou au-dessus des renflements latéraux (15,16 ; 21,22) des tubes d'échangeur (17,18).

5. Réacteur selon la revendication 1 et l'une des revendications 2 à 4, caractérisé en ce que les collecteurs et distributeurs (7,8 ou 37,38) sont cintrés et en ce qu'un tube (7,8 ou 37,38) est disposé dans une moitié de section transversale du réacteur (1), les tubes 7,8 ou 37,38) étant disposés symétriquement par rapport au plan médian transversal du réacteur.

6. Réacteur selon la revendication 1, caractérisé en ce que les sections (28) de fond consistent en éléments individuels (27), qui peuvent basculer à l'aide d'arbres individuels (26) montés sur les tubes porteurs inférieurs (23).

7. Réacteur selon la revendication 6, caractérisé en ce que la face avant et la face arrière (29,31) des éléments individuels (27) de fond, sont pourvues de saillies (32) en forme de crochet et de ressauts correspondants (30), qui s'interpénètrent quand le fond est fermé.

8. Réacteur selon les revendications 6 et 7, caractérisé en ce que les sections (28a,28b) de fond disposées des deux côtés du plan vertical médian du réacteur (W) peuvent basculer en sens opposés.

9. Réacteur selon la revendication 8, caractérisé en ce que les deux sections (28a,28b) de fond disposées des deux côtés du plan vertical médian du réacteur (W) et parallèlement aux arbres individuels (26) des sections (28) s'appuient sur des corps d'appui (46) communs pouvant basculer.

10. Réacteur selon la revendication 9, caractérisé en ce que les corps d'appui (46) disposés au moins à la distance des sections de fond (28a,28b) de chaque côté du plan médian du réacteur (W) peuvent être basculés par un arbre commun (45) dans le plan médian vertical du réacteur et présentent des surfaces d'appui (47,48), sur lesquelles viennent reposer des surfaces d'appui (49,50) des sections (28a,28b) de fond.

11. Réacteur selon la revendication 10, caractérisé en ce que les extrémités inférieures des surfaces d'appui (49,50) des sections (28a,28b) de fond sont pourvues de saillies ou d'ergots (51,52).

12. Réacteur selon les revendications à 11, caractérisé en ce que l'arbre (45) entre les sections (28a,28b) de fond est recouvert par un élément (56) en forme de toit.

Fig.1

Fig. 2

Fig.3

Fig.4

Fig.6

Fig.5

Fig.7

EP 0 216 152 B1